# EUROPEAN PATENT SPECIFICATION

(11) **EP 3 820 446 B1**
(45) Date of publication and mention of the grant of the patent: **03.11.2021**
(21) Application number: 19790718.1
(22) Date of filing: 09.10.2019
(51) Int. Cl.: A61K 9/00, A61K 9/10, A61K 31/519, A61K 47/06, A61P 11/08

(54) **PHARMACEUTICAL COMPOSITIONS COMPRISING RPL554 IN HFA-134A FOR ADMINISTRATION BY INHALATION**
PHARMAZEUTISCHE ZUSAMMENSETZUNGEN, UMFASSEND RPL554 IN HFA-134A ZUR VERABREICHUNG DURCH INHALATION
COMPOSITIONS PHARMACEUTIQUES COMPRENANT DU RPL554 DANS DU HFA-134A POUR UNE ADMINISTRATION PAR INHALATION

(30) Priority: 09.10.2018 GB 201816447
(43) Date of publication of application: 19.05.2021
(60) Divisional application: 21198029.7
(73) Proprietor: Verona Pharma PLC, Cardiff CF10 1FS (GB)
(72) Inventor: SPARGO, Peter Lionel, Canterbury Kent CT2 8NR (GB); HAYWOOD, Phillip A, Buntingford Hertfordshire SG9 0DH (GB)
(74) Representative: J A Kemp LLP
(86) International application number: PCT/GB2019/052863
(87) International publication number: WO 2020/074894

(56) References cited:
- WO-A1-2014/140647
- WO-A1-2015/173551
- WO-A1-2016/128742

## Description

### FIELD OF THE INVENTION

The present invention relates to a liquid pharmaceutical composition comprising a respiratory drug and a pressurised metered dose inhaler (pMDI) comprising the liquid pharmaceutical composition.

### BACKGROUND OF THE INVENTION

RPL554 (9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one) is a dual PDE3/PDE4 inhibitor and is described in WO 00/58308. As a combined PDE3/PDE4 inhibitor, RPL554 has both anti-inflammatory and bronchodilatory activity and is useful in the treatment of respiratory disorders such as asthma and chronic obstructive pulmonary disease (COPD). The structure of RPL554 is shown below.

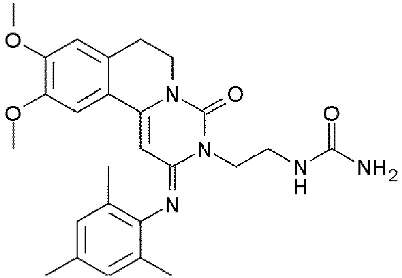

RPL554 is typically administered by inhalation in view of its efficacy in the treatment of respiratory disorders. Administration of RPL554 by nebulizer is known (WO 2016/042313). However, it is often desirable to administer respiratory drugs using a pressurised metered dose inhaler (pMDI).

pMDI formulations comprising salts of RPL554 are envisaged in WO 2015/173551. A pMDI formulation comprising a combination of RPL554 with a second active agent and a surfactant is described in WO 2014/140648. WO 2016/128742 A1 discloses pMDI formulations comprising RPL554 as free base and as salts and HFA 134a. WO 2014/140647 A1 discloses the preparation of a pMDI composition where a micronized RPL554 combination was dispersed in a solution comprising lecithin in deionised water (200 mL). The resulting suspension was spray dried, micronized and then loaded into MDI cartridges containing pressurised 1,1,1,2-tetrafluoroethane.

Known pMDI formulation strategies can involve the use of a wide range of different propellants, excipients, co-solvents and surfactants, and may take the form of suspensions, solutions or mixtures thereof. For instance, pMDI formulations may include one or more propellants such as alkanes (for instance propane, n-butane, isobutane, n-pentane, isopentane, neopentane), hydrofluoroalkanes (HFAs, for instance HFA-227, HFA-134a and HFA 152a), ethers (for instance dimethylether) and hydrofluoro-olefins (HFOs, for instance HFO-1234ze and HFO-1234yf). Co-solvents such as ethanol and water are commonly included in pMDI formulations. Excipients which are commonly included in pMDI formulations include antioxidants, preservatives, wetting agents, chelating agents, emulsifiers, flavourings, buffers, lubricants, suspending agents and tonicity adjusting agents.

Drugs which have low solubility in diluents are often formulated as suspensions. In such suspension formulations, an important consideration is uniform dispersion of the drug particles. Agglomeration, phase separation and flocculation can lead to variability in the metered dose. Additional excipients such as surfactants and co-solvents are therefore commonly used in suspension formulations to improve suspension properties. A further consideration for suspension formulations is that of particle size distribution. The particle size distribution of the drug is usually maintained in the respirable range (typically less than 5 µm).

Each of the components included in pMDI can have a number of effects on formulation stability and efficacy which are highly dependent on the identity of the drug to be formulated. Such effects cannot reliably be predicted in advance. Correct formulation of a drug for use in a pMDI is critical to ensure that the formulation can be used successfully in a clinical setting.

### SUMMARY OF THE INVENTION

It is a surprising finding of the present invention that a liquid pharmaceutical composition comprising particles of RPL554 and HFA-134a (1,1,1,2-tetrafluoroethane) as a diluent/propellant and which is substantially free of a surfactant is particularly advantageous for delivery of RPL554 by a pressurised metered dose inhaler. A number of benefits have been found to be associated with the liquid pharmaceutical composition. The specific combination of RPL554 and HFA-134a has been found to lead to a suspension pMDI formulation which has favourable suspension characteristics and reduced flocculation. It has also been found that a chemically and physically stable formulation may be achieved without requiring the presence of surfactants.

The ability to omit surfactants from the composition for use of the invention is highly advantageous. That is because RPL554 is likely to be used in patients suffering from conditions such as COPD and asthma, who are at increased risk of an allergic inflammatory reaction to excipients such as surfactants. Further, given the teaching in WO 2014/140648 which suggests that a surfactant is necessary to achieve an adequate pMDI, it is surprising that adequate long term stability, without aggregation or flocculation of the particles of active ingredient, can be achieved without a surfactant.

It has also unexpectedly been found that particles of RPL554 are vulnerable to Ostwald ripening (growth of particles during suspension) in the presence of co-solvents such as ethanol. It is important to avoid Ostwald ripening as an increase in particle size can reduce the respirable fraction of particles present in the formulation. The liquid pharmaceutical formulations for use of the invention are found to avoid Ostwald ripening of the RPL554 particles.

The present invention provides a liquid pharmaceutical composition for use in the treatment or prevention of chronic obstructive pulmonary disease (COPD), which liquid pharmaceutical composition is suitable for administration by inhalation and comprises: (i) a suspension of particles comprising 9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one (RPL554); and (ii) a diluent which is 1,1,1,2-tetrafluoroethane (HFA-134a), wherein the liquid pharmaceutical composition contains less than 0.05 % by weight of a surfactant relative to the total weight of the composition.

The invention further provides a pressurised metered dose inhaler comprising a liquid pharmaceutical composition for use according to the invention.

### BRIEF DESCRIPTION OF THE FIGURES

Figure 1 shows a schematic of visual assessment of suspension performance in pressure rated glass vials.
Figure 2 shows the sedimentation score against propellant mixtures for each product strength.
Figure 3 shows a diagrammatic representation of a test canister after 24 hours with the dip tube location into the liquid phase.
Figure 4 shows a RPL554 solubility graph with increasing ethanol concentrations, with or without oleic acid.
Figure 5 shows mean fine particle dose against product strength of the pMDI formulations.

### DETAILED DESCRIPTION OF THE INVENTION

The liquid pharmaceutical composition for use of the invention comprises a suspension of particles of 9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one (RPL554). For the avoidance of doubt, the RPL554 is in free-base form. While the majority of the particles present in the liquid pharmaceutical composition are typically in suspension in the diluent, it is also the case that some or all of the particles in the liquid pharmaceutical composition for use according to the invention may have settled to the bottom of a receptacle containing the liquid pharmaceutical composition, for instance after storage for a period of time. The particles may be resuspended in any suitable way, for instance by agitation of the liquid pharmaceutical composition (for instance by shaking a canister comprising the liquid pharmaceutical composition).

The diluent in the liquid pharmaceutical composition is 1,1,1,2-tetrafluoroethane which is known as HFA-134a and has the formula CH₂FCF₃. The diluent also acts as the propellant. Typically, HFA-134a is the sole diluent in the liquid pharmaceutical composition. In another embodiment, the liquid pharmaceutical composition comprises a diluent which comprises greater than 90 wt% of HFA-134a relative to the total weight of diluent in the liquid pharmaceutical composition. The diluent may comprise greater than 95 wt% of HFA-134a, greater than 98 wt% or greater than 99.5 wt% of HFA-134a, relative to the total weight of diluent in the liquid pharmaceutical composition. The diluent may consist essentially of HFA-134a. A composition which consists essentially of a component typically comprises only that component and any other components which do not materially affect the essential characteristics of the component of which the composition essentially consists. Typically, the diluent consists of HFA-134a. The diluent corresponds to the liquid component of the liquid pharmaceutical composition.

The liquid pharmaceutical composition contains less than 0.05 % by weight of a surfactant. As used herein, a composition is "substantially free" of a specified component if it contains less than 0.5 % by weight, preferably less than 0.1 % by weight, more preferably less than 0.01 % by weight of the specified component relative to the total weight to the composition, for instance less than 0.001 % by weight of the specified component. Typically, the liquid pharmaceutical composition does not comprise a surfactant.

Examples of surfactants include non-ionic surfactants, anionic surfactants, cationic surfactants or zwitterionic surfactants. For the avoidance of doubt, the liquid pharmaceutical composition is therefore substantially free of lecithin, oleic acid, polyvinyl pyrrolidone K25, polyvinyl alcohol, oligolactic acid, sodium dioctyl sulfosuccinate, polyoxyethylene glycol alkyl ethers (for instance PEG 300, PEG 600, PEG 1000, Brij 30, Brij 35, Brij 56, Brij 76 and Brij 97), polypropylene glycol (for instance PPG 2000), glucoside alkyl ethers, polyoxyethylene glycol octylphenol ethers, polyoxyethylene glycol alkylphenol ethers, glycerol alkyl esters, polyoxyethylene glycol sorbitan alkyl esters (polysorbates, for instance polysorbate 20, polysorbate 60 and polysorbate 80), sorbitan alkyl esters (for instance sorbitan monolaurate (Span 20), sorbitan monooleate (Span 80) and sorbitan trioleate (Span 85)), cocamide MEA, cocamide DEA, dodecyldimethylamine oxide, block copolymers of polyethylene glycol and polypropylene glycol (poloxamers), block copolymers of polyethylene glycol and polypropylene oxide (for instance Pluronic surfactants) and polyethoxylated tallow amine (POEA).

In a preferred embodiment, the liquid pharmaceutical composition for use of the invention is substantially free of ethanol.

More preferably, the liquid pharmaceutical composition is substantially free of any additional excipient selected from a co-solvent and a surfactant. The liquid pharmaceutical composition more typically does not comprise any co-solvent and does not comprise any surfactant.

Examples of co-solvents include ethanol, pentane, water, isopropanol, glycerol and propylene glycol.

In a preferred embodiment, the liquid pharmaceutical composition is substantially free of any additional excipient. The additional excipient may for example be a surfactant or co-solvent as described above, or an excipient selected from antioxidants, preservatives, wetting agents, solubilizers, emulsifiers, flavouring agents, chelating agents, humectants, tonicity adjusting agents, pH adjusters, dispersion agents and suspending aids.

The liquid pharmaceutical composition may for instance contain less than 0.01 % or less than 0.001 % by weight of said surfactant or said additional excipient, relative to the total weight of the composition.

Typically, at least 50% by weight, more preferably at least 90% by weight, more preferably at least 99% by weight of the active pharmaceutical ingredient (API) in the liquid composition for use of the invention is RPL554, based on the total weight of all APIs.

More typically, RPL554 is the sole active agent in the liquid pharmaceutical composition. In this embodiment, the liquid pharmaceutical composition for use of the invention is substantially free from other active agents (for instance a muscarinic receptor antagonist or a beta-adrenergic receptor agonist).

As explained above, it has been found that a liquid pharmaceutical composition comprising HFA-134a and particles of RPL554 and which is substantially free of any additional excipient has a number of advantages. Typically, therefore, the liquid pharmaceutical composition consists essentially of (i) RPL554 and (ii) 1,1,1,2-tetrafluoroethane. More typically, the liquid pharmaceutical composition comprises at least 99.5 % by weight of (i) RPL554 and (ii) 1,1,1,2-tetrafluoroethane relative to the total weight of the composition. The liquid pharmaceutical composition may for instance consist of (i) RPL554 and (ii) 1,1,1,2-tetrafluoroethane.

A liquid pharmaceutical composition for use according to the invention may for instance comprise: (i) RPL554 in an amount of 0.005 to 5 % by weight and (ii) 1,1,1,2-tetrafluoroethane (HFA-134a) in an amount of from 95 to 99.995 % by weight, wherein the weight % is relative to the total weight of the liquid pharmaceutical. Typically the total amount of RPL554 and HFA-134a is at least 99 % by weight relative to the total weight of the composition.

The particles of RPL554 may be of any suitable size for a use in a liquid pharmaceutical composition suitable for inhalation. Typically, the particles of RPL554 are micronized particles. For instance, the particles of RPL554 may have a Dv50 (median particle size by volume) value of less than or equal to 10 µm or from about 0.1 µm to about 8 µm.

Typically, the particles have a particle size distribution with a Dv50 value of from about 0.2 µm to about 5 µm. More typically, the particles of RPL554 have a particle size distribution with a Dv50 value of from about 0.7 µm to about 3.0 µm. For instance, the particles of RPL554 may have a particle size distribution with a Dv50 value of from 0.9 µm to 2.7 µm Often, the particles of RPL554 have a particle size distribution with a Dv50 value of from about 1.1 µm to about 2.1 µm.

The Dv50 value is the median particle size for a volume distribution. Thus, half the volume of the particles is comprised in particles having diameters of less than the Dv50 value and half the volume of the particles is comprised in particles having diameters of greater than the Dv50 value. This is a well known manner in which to describe particle size distributions.

The particles typically have a particle size distribution with a Dv10 value of from about 0.4 µm to about 1.0 µm. The particles typically have a particle size distribution with a Dv90 value of from about 2.0 µm to about 4.0 µm. The Dv10 value reflects the particle diameter where 10% of the volume of the sample is in particles having a particle diameter less than the Dv10 value. The Dv90 value reflects the particle diameter where 90% of the volume of the sample is in particles having a particle diameter less than the Dv90 value.

The technique used to measure the Dv50 value is typically laser diffraction. For instance, the RPL554 particles typically have a particle size distribution with a Dv50 value of from about 0.2 µm to about 5 µm as measured by laser diffraction. The particle size distribution analysis can be performed by laser diffraction using the Malvern Spraytec in conjunction with a wet dispersion cell. Typically, the instrument parameters for the Malvern Spraytec are as follows:
- particle ― standard opaque particle;
- refractive index Particle ― 1.50;
- refractive index (imaginary) ― 0.50;
- density of particle ― 1.00;
- refractive index of dispersant ― 1.33;
- controller unit ― 1000RPM;
- measurement type ― timed;
- initial sampling time ― 30s;
- obscuration ― 20% - 30%;
- dispersant ― 1% Polysorbate 20 in deionised water.

The particles of RPL554 may be produced by any pharmaceutically acceptable size reduction process or particle size controlled production process. For instance, the particles may be produced by spray-drying a solution of RPL554, by controlled crystallisation, or by size reduction of a solid form of RPL554, for example by air jet milling, mechanical micronisation or media milling.

The concentration of the particles comprising RPL554 in the liquid pharmaceutical composition may be varied based on the intended dosage of the active compound per actuation of an inhaler. For instance, the concentration of particles may be such that the dose of RPL554 delivered per actuation of an inhaler comprising the liquid pharmaceutical composition is from 5 µg/actuation to 1500 µg/actuation. The concentration of particles may be such that the dose of RPL554 delivered per actuation from 50 µg/actuation to 1000 µg/actuation.

The concentration of the particles comprising RPL554 in the liquid pharmaceutical composition may be from about 0.01 mg/mL to about 400 mg/mL. The concentration of the particles comprising RPL554 in the liquid pharmaceutical composition is typically from about 0.1 mg/mL to about 200 mg/mL. More typically, the concentration of particles of RPL554 in the liquid pharmaceutical composition is from about 0.5 mg/mL to about 20 mg/mL.

For instance, the concentration of particles of RPL554 in the liquid pharmaceutical composition may be from about 0.1 mg/mL to about 3.0 mg/mL or from about 0.5 mg/mL to about 2.0 mg/mL. The concentration of particles of RPL554 in the liquid pharmaceutical composition may be from about 5.0 mg/mL to about 20 mg/mL or from about 6.0 mg/mL to about 10 mg/mL.

The concentration of the particles comprising RPL554 in the liquid pharmaceutical composition may be from about 0.01 % w/w to about 5.0 % w/w. The concentration of the particles comprising RPL554 in the liquid pharmaceutical composition is typically from about 0.05 % w/w to about 2.0 % w/w.

The liquid pharmaceutical composition is typically suitable for administration by inhalation. The liquid pharmaceutical composition is more typically suitable for administration by pressurised metered dose inhaler. A pressurised metered dose inhaler is an inhaler which delivers an aerosolised dose of a liquid pharmaceutical composition using a pressurised liquefied propellant. A pressurised metered dose inhaler typically comprises a canister (or vial) which comprises a liquid pharmaceutical composition, a metering valve, an actuator and a mouthpiece.

The invention provides a pressurised metered dose inhaler comprising a liquid pharmaceutical composition for use as defined herein. pMDIs are well known to those of ordinary skill in the art, and many such devices are commercially available, with representative devices including AeroBid Inhaler System (Forest Pharmaceuticals), Atrovent Inhalation Aerosol (Boehringer Ingelheim), Flovent^{RTM} (GlaxoSmithKline), Maxair Inhaler (3M), Proventil^{RTM} Inhaler (Schering) and Serevent^{RTM} Inhalation Aerosol (GlaxoSmithKline).

The pressurised metered dose inhaler comprising a liquid pharmaceutical composition may be configured to provide upon actuation an aerosol of the liquid pharmaceutical composition which has a median mass aerodynamic diameter (MMAD) of from 1.0 µm to 5.0 µm. The MMAD may be as measured using the Next Generation Impactor as per compendial requirements (European Pharmacopoeia (Ph. Eur.) Method Chapter 2.9.18 & & US Pharmacopeia Chapter <601>).

Typically, the liquid pharmaceutical composition is administered by inhalation.

The liquid pharmaceutical composition is for use in the treatment or prevention of chronic obstructive pulmonary disease (COPD).

Typically, the liquid pharmaceutical composition is administered by inhalation with a metered nominal dose of from 5 µg to 1000 µg per actuation from a pMDI comprising the liquid pharmaceutical composition. For instance, the metered nominal dose per actuation may be from 10 µg to 500 µg. Typically, two actuations of the pMDI may be inhaled in succession to provide a single dose.

Typically, the emitted dose from a pMDI comprising the liquid pharmaceutical composition is from 80% to 95% of the metered nominal dose.

Typically, the liquid pharmaceutical composition is administered to the patient via 2 to 8 actuations of the pMDI per day.

The invention is described in more detail by the following Examples.

### EXAMPLES

### Materials and methods

The materials used are as follows.
• API: Micronised RPL554. The properties of the micronised RPL554 are shown in Table 1 below.

**Table 1**

| Property | Description |
|---|---|
| Molecular formula | C₂₆H₃₁N₅O₄ |
| Molecular weight | 477.56 g/mol |
| Physical form | yellow crystalline solid |
| DSC characteristics | onset: 247.83°C |
| | peak: 250.21°C |

• Propellant: HFA-134a and HFA-227ea.
• Co-solvent: Ethyl alcohol/dehydrated alcohol (100% USP-NF ethanol), obtained from Hyman Kimia.
• Surfactant: Oleic acid (Ph. Eur./BP) obtained from Croda International plc.
• Reagents: Analytical grade chemicals were used throughout the study.
• Borosilicate glass vials with a metal foil lined polypropylene closure (VWR, Part number 215-3905).
• Glass vials: 15 ml pressure rated clear glass vials (Neville & Moore, Part No 0771C3/A3).
• Valves: Bespak and Aptar 63 µl valves.
• Cans: Presspart 14 ml can.
• Actuators: Presspart actuator.

All samples were prepared in the laboratory using a Pamasol hand crimper and filler. All samples were mixed by immersion in an ultrasonic bath for 30 seconds.

All analytical methods were fully developed and in vitro testing was carried out using DUSA tubes and by Next Generation Impaction (NGI).

The analytical test methods used during analysis are as follows.
- Delivered Dose testing performed as per compendial requirements (European Pharmacopoeia (Ph. Eur)- Preparations for Inhalation (0671) & US Pharmacopeia Chapter <601>)
- Aerodynamic Particle Size Distribution (APSD) testing performed using the Next Generation Impactor as per compendial requirements (European Pharmacopoeia (Ph. Eur.) Method Chapter 2.9.18 & & US Pharmacopeia Chapter <601>)

### Example 1 ― Assessment of suitable propellants

Experiments were conducted to compare the suitability of two propellants, HFA-134a and HFA-227ea, for use as diluents for a pMDI suspension formulation comprising RPL554. Table 2 shows the key physical and chemical properties of the two propellants evaluated.

**Table 2 - HFA Propellant Properties**

| | HFA-134a | HFA-227ea |
|---|---|---|
| Chemical Name | 1,1,1,2-tetrafluoroethane | 1,1,1,2,3,3,3-heptafluoroethane |
| Chemical formula | CF₃-CH₂F | CF₃-CFH-CF₃ |
| Molar mass (g/mol) | 102.03 | 170.03 |
| Boiling point at 1.1013 bar (°C) | -26.3 | -16.5 |
| Freezing point (°C) | -101 | -131 |
| Density at 20°C (kg/dm³) | 1.226 | 1.408 |
| Dynamic viscosity of the liquid at 20°C (mPa.s) | 0.211 | 0.267 |
| Surface tension of the liquid at 20° (mN/m) | 8.69 | 6.96 |

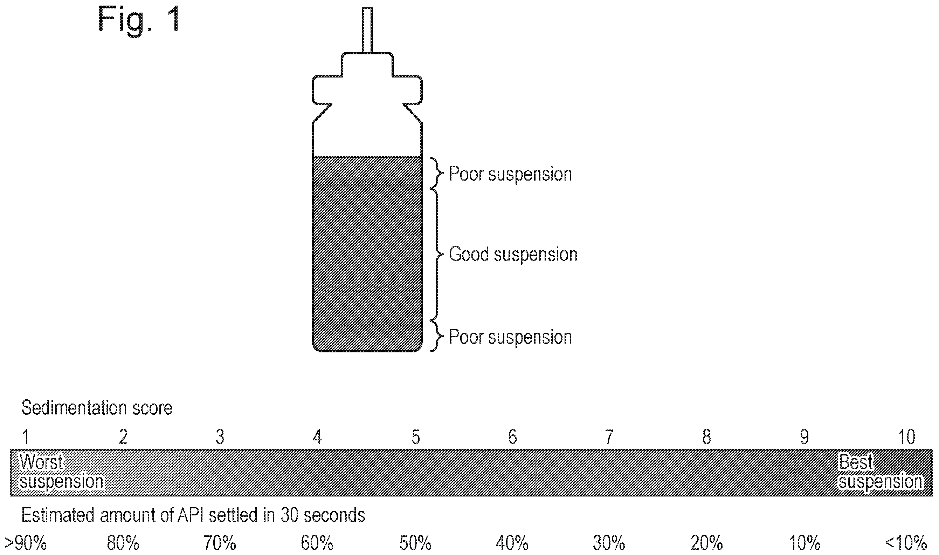

Samples were made by adding the required amounts of RPL554 (see Table 3) to 15 ml pressure rated clear glass vials. The propellant, HFA-134a, HFA-227ea or combinations of the two (see Table 4) was added through the valve and the samples were mixed in an ultrasonic bath for 30 seconds. The same target fill weight of 11.6 g (±0.5 g) was used for all samples. The appearance of the API particles in the propellants was assessed with regards to both the sedimentation rate and flocculation behaviour (see Figure 1). The sedimentation rate of the formulations was visually assessed after suspension agitation by recording images of the glass vials using a high-speed camera. The suspension quality was determined by assigning a sedimentation score of from 1 to 10 based on the amount of active particles which had settled or floated in 30 seconds, with 1 being the fastest to sediment/cream (i.e. undesirable) and 10 being the slowest to sediment/cream (i.e. acceptable).

**Table 3 - Quantities of RPL554 used for formulations of HFA-134a, HFA-227ea and mixtures thereof**

| Product strength (µg/actuation) | Mass of RPL554 (mg/vial) |
|---|---|
| 10 | 1.5 |
| 50 | 7.5 |
| 150 | 22.5 |
| 500 | 75.0 |
| 1000 | 150.0 |

**Table 4 - Propellant ratios for each formulation**

| HFA-134a (% w/w) | HFA-227ea (% w/w) |
|---|---|
| 100 | 0 |
| 75 | 25 |
| 50 | 50 |
| 25 | 75 |
| 0 | 100 |

### Results

The RPL554 formulation in HFA-134a was readily suspended on shaking, with rapid flocculation and gradual sedimentation observed after shaking was stopped. The formulations in HFA-227ea and blends of HFA-134a/HFA-227ea (50/50) were also readily suspended on shaking. However, creaming was observed after the cessation of shaking for both HFA-227ea containing formulations. The HFA-227ea formulations showed evidence of particle adhesion to the container walls together with more distinct, larger flocs after shaking.

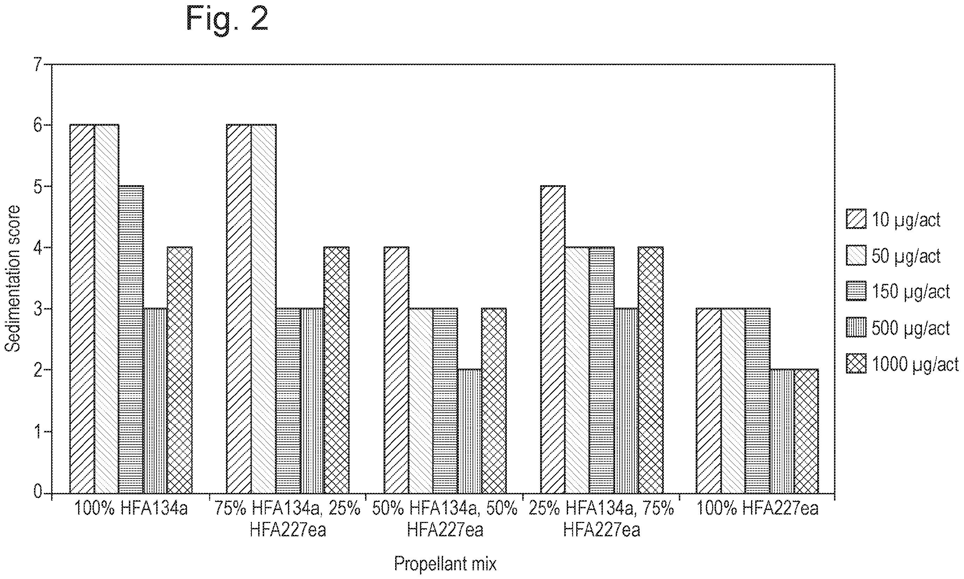

The sedimentation scores using the propellant (pure or mixture) for each product strength are shown graphically in Figure 2. This shows that the best score (i.e. the slowest sedimentation) was observed in formulations with higher HFA-134a content. The sedimentation score decreased with increased product strength owing to the increase in powder loading. Table 5 shows the individual sedimentation scores.

**Table 5 - Sedimentation scores for formulations prepared with HFA-134a and HFA-227ea and mixtures thereof**

| Product strength (µg/actuation) | HFA-134a % w/w | HFA-227ea % w/w | Score |
|---|---|---|---|
| 10 | 100 | 0 | 6 |
| 50 | | | 6 |
| 150 | | | 5 |
| 500 | | | 3 |
| 1000 | | | 4 |
| 10 | 75 | 25 | 6 |
| 50 | | | 6 |
| 150 | | | 3 |
| 500 | | | 3 |
| 1000 | | | 4 |
| 10 | 50 | 50 | 4 |
| 50 | | | 3 |
| 150 | | | 3 |
| 500 | | | 2 |
| 1000 | | | 3 |
| 10 | 25 | 75 | 5 |
| 50 | | | 4 |
| 150 | | | 4 |
| 500 | | | 3 |
| 1000 | | | 4 |
| 10 | 0 | 100 | 3 |
| 50 | | | 3 |
| 150 | | | 3 |
| 500 | | | 2 |
| 1000 | | | 2 |

As can be seen from Table 5, it was unexpectedly found that formulations comprising HFA-134a as a diluent had consistently improved sedimentation scores compared with formulations comprising HFA-227ea as a diluent. Formulations comprising a higher percentage of HFA-134a were found to lead to less flocculation, less creaming and less adhesion compared with formulations comprising a higher percentage of HFA-227ea.

It has therefore been discovered that HFA-134a may advantageously be used as the sole diluent for a liquid pharmaceutical composition comprising a suspension of particles of RPL554.

### Example 2 - Assessment of suitable additional excipients

In order to seek to improve the suspension characteristics the inclusion of additional excipients was investigated. In particular, formulations further comprising a surfactant (oleic acid, Ph. Eur./BP) and a co-solvent (ethanol, 100% USP-NF) were prepared. The compositions of the formulations tested and the observed sedimentation scores are shown in Table 6.

**Table 6 - Sedimentation scores of pMDI formulations containing HFA-134a, oleic acid and ethanol in varying amounts**

| Product strength (µg/actuation) | Ethanol % w/w | Oleic acid % w/w | Score |
|---|---|---|---|
| 10 | 1 | 0.010 | 6 |
| | 1 | 0.500 | 1 |
| | 5 | 0.255 | 6 |
| | 15 | 0.010 | 6 |
| | 15 | 0.500 | 6 |
| 150 | 1 | 0.255 | 2 |
| | 5 | 0.010 | 4 |
| | 5 | 0.255 | 5 |
| | 5 | 0.255 | 5 |
| | 5 | 0.255 | 4 |
| | 5 | 0.255 | 5 |
| | 5 | 0.255 | 5 |
| | 5 | 0.255 | 5 |
| | 5 | 0.500 | 4 |
| | 15 | 0.255 | 8 |
| 1000 | 1 | 0.010 | 3 |
| | 1 | 0.500 | 2 |
| | 5 | 0.255 | 3 |
| | 15 | 0.010 | 4 |
| | 15 | 0.500 | 5 |

It was observed that the suspension scores for the formulations comprising oleic acid and ethanol were generally similar to or lower than those formulations which are free from such excipients. However, in some samples it was observed that the RPL554 appeared to have dissolved and recrystallized. A solubility analysis was conducted to assess the extent of dissolution of RPL554 in the additional excipients.

### Example 3 - RPL554 solubility in ethanol

RPL554 was originally deemed to be practically insoluble in ethanol, according to USP/BP solubility criteria presented in Table 7. However, the observations of dissolution and recrystallisation with the lowest product strength (10 µg/actuation) prompted further investigations into the solubility of RPL554 in ethanol in the presence of oleic acid.

**Table 7 - USP and BP solubility criteria**

| Descriptive term | Part of solvent required per part of solute |
|---|---|
| Very soluble | less than 1 |
| Freely soluble | from 1 to 10 |
| Soluble | from 10 to 30 |
| Sparingly soluble | from 30 to 100 |
| Slightly soluble | from 100 to 1000 |
| Very slightly soluble | from 1000 to 10,000 |
| Practically insoluble | more than 10,000 |

### Experimental method

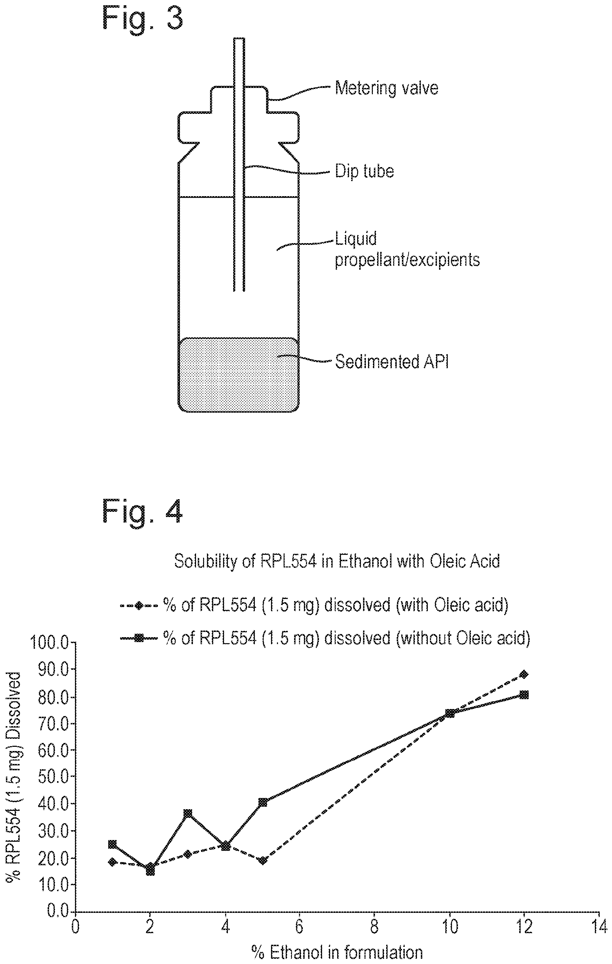

Samples were prepared by adding 1.5 mg of RPL554 to plain 14 mL aluminium canisters, with ethanol, oleic acid and HFA-134a. Excipients were added to the canisters to maintain an ethanol/oleic acid ratio of a 50:1 (see Table 8). Corresponding canisters were also produced containing RPL554, ethanol and HFA-134a only. The canisters were crimped with 63 µL pMDI valves, with shortened dip tubes (Figure 3), and filled to 11.6 g with HFA-134a through the valve. The length of the dip tubes was sufficient to extend into the liquid propellant/co-solvent phase only (i.e. after the API particles had settled). The samples were placed in an ultrasonic bath for 30 seconds.

**Table 8 - Excipient concentrations used in the assessment of RPL554 solubility in ethanol**

| Product strength (µg RPL554/actuation) | Ethanol % w/w | Oleic acid % w/w |
|---|---|---|
| 10 µg | 1 | 0.02 |
| | 2 | 0.04 |
| | 3 | 0.06 |
| | 4 | 0.08 |
| | 5 | 0.10 |
| | 10 | 0.20 |
| | 12 | 0.24 |

All the canisters were left for 24 hours at ambient temperature to settle and to establish dissolution equilibrium. To determine the amount of API dissolved, each canister was fired five times, in an upright position (through a cannula fitted to the valve stem) into a 10.0 cm³ volumetric flask. The flask was made up to volume with dehydrated ethanol and the solution was analysed using UV/Visible spectroscopy (Perkin Elmer, Model Lambda 35). The concentration of RPL554 was calculated, quantitatively, against a three point standard curve.

### Results

The amount of dissolved RPL554 increased with increasing ethanol concentration. The presence of oleic acid in the formulation appeared to have no effect on the solubility of RPL554 (Figure 4).

Previous assessment of the solubility of RPL554 in ethanol led to a classification of "practically insoluble" according to the USP/BP criteria. The original assessment was carried out using Analytical Reagent grade ethanol. A reassessment of the solubility, this time using dehydrated alcohol (100% USP-NF ethanol, i.e. the grade of ethanol commonly used in bulk pMDI HFA formulation suspensions manufacture) found the solubility of RPL554 in ethanol to be approximately 1 part in 2000, which is "very slightly soluble" according to the USP/BP criteria. Unexpectedly, RPL554 was therefore found to have some solubility in ethanol.

For suspension formulations, the solubility of the API in the co-solvent/propellant mix should be minimal to avoid potential issues with chemical and physical instability. Any benefits associated with the use of oleic acid and ethanol as excipients was found to be significantly outweighed by the vulnerability towards dissolution of RPL554 and Ostwald ripening of the RPL554 particles. It was therefore established that the most promising pMDI formulation for RPL554 was a suspension formulation of RPL554 in HFA-134a only.

### Example 4 - Validation of HFA-134a only formulation

The HFA-134a only formulations were further assessed to ensure that the RPL554 was sufficiently suspended for delivery via a pMDI, by preparing samples in pressure rated glass vials. Three product strengths (50, 150 and 500 µg/actuation) were selected. Visual assessment of these formulations indicated comparable suspension quality and sedimentation rates to commercially available HFA-134a only pMDI product.

The product performance attributes of the RPL554 HFA-134a only suspension based formulations were further assessed by preparing laboratory samples in 14 mL plain aluminium canisters, at 3 strengths (50, 150 and 500 µg/actuation), which were crimped with Aptar 63 µL valves. The samples were mixed for 30 seconds in an ultrasonic bath before being stored, inverted, for 4 days prior to analysis for Delivered Dose Uniformity (DDU) and Aerodynamic Particle Size Distribution (APSD) by Next Generation Impaction (NGI).

### Delivered Dose Uniformity (DDU)

Delivered dose uniformity analysis was carried at the beginning, middle and end of canister life in triplicate on three samples of each product strength. The mean delivered dose results are shown in Table 9.

Overall, the mean delivered dose was close to the anticipated target label claim for each product strength.

### Shot Weight

The shot weight was measured at the beginning, middle and end of canister life to assess product performance through life.

The shot weight for the laboratory prepared samples was found to be consistent across all samples with a mean value of 80.35 ± 0.54 mg (RSD = 0.67%, n = 9). The theoretical shot weight for 63 µL of HFA-134a is 77.2 mg.

### Aerodynamic Particle Size Distribution (APSD)

The aerodynamic particle size distribution was measured in order to determine the fine particle fraction (Fine particle fraction, % dose with particle size of less than 5 µm) of the tested formulations. The results are shown in Table 9.

**Table 9 - Summary of the Aerodynamic Particle Size Distribution and Delivered Dose for RPL554 HFA-134a pMDI**

| | | **50 µg** | **150 µg** | **500 µg** |
|---|---|---|---|---|
| **APSD attributes by NGI** | **Fine Particle Dose (µg <5 µm)** | 15.81 | 58.89 | 171.74 |
| | **Fine Particle Fraction (%)** | 42.45 | 43.67 | 46.14 |
| | **MMAD (µm)** | 1.91 | 1.96 | 2.97 |
| | **GSD** | 1.88 | 1.91 | 1.95 |
| **DDU** | **Mean Delivered Dose (µg/actuation)** | 41.87 | 145.59 | 493.31 |

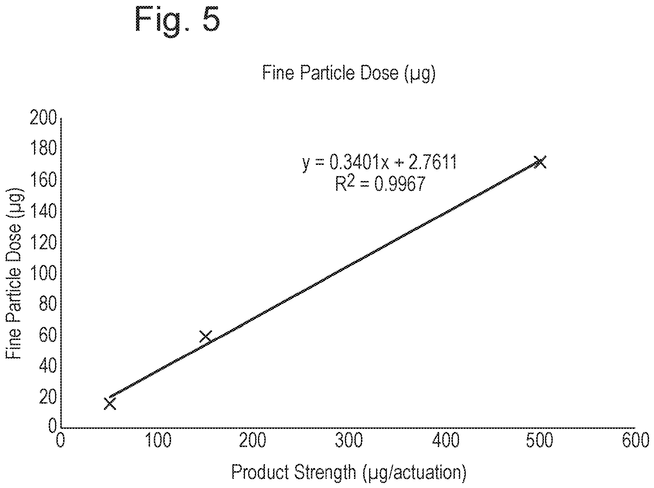

The correlation between the determined fine particle dose (FPD) and product strength is shown in Figure 5. Dose proportionality was observed.

### Example 5 ― Formulation Stability

Stability studies were performed on all RPL554 formulation strengths. Example data are presented for RPL554 HFA-134a pMDI, 500 µg/actuation product in Table 10 and Table 11 for stability studies conducted at 25°C/60 and 40°C/75% RH respectively. The data show consistent performance over the 6 month stability duration.

**Table 10 - RPL554 HFA-134a pMDI, 500 µg/actuation ― Stability Data for 25°C/60% RH Storage Condition**

| **Quality Attribute** | **Timepoint** | | |
|---|---|---|---|
| | **Initial** | **3 months** | **6 months** |
| Total Drug Content (% w/w) | 0.642% | 0.648% | 0.639% |
| Total Impurities (as % LC) | 0.64% | 0.65% | 0.59% |
| Mean Delivered Dose (µg/actuation) | 440.4 | 445.2 | 476.0 |
| Fine Particle Dose (µg < 5 µm) | 171.7 | 164.2 | 172.8 |

**Table 11 - RPL554 HFA-134a pMDI, 500 µg/actuation ― Stability Data for 40°C/75% RH Storage Condition**

| **Quality Attribute** | **Timepoint** | | | |
|---|---|---|---|---|
| | **Initial** | **1 month** | **3 months** | **6 months** |
| Total Drug Content (% w/w) | 0.642% | 0.635% | 0.646% | 0.641% |
| Total Impurities (as % LC) | 0.64% | 0.63% | 0.64% | 0.67% |
| Mean Delivered Dose (µg/actuation) | 440.4 | 472.9 | 462.3 | 481.0 |
| Fine Particle Dose (µg < 5 µm) | 171.7 | 159.6 | 175.5 | 177.4 |

## Claims

1. A liquid pharmaceutical composition for use in the treatment or prevention of chronic obstructive pulmonary disease (COPD),
which liquid pharmaceutical composition is suitable for administration by inhalation and comprises:
(i) a suspension of particles comprising 9,10-dimethoxy-2-(2,4,6-trimethylphenylimino)-3-(N-carbamoyl-2-aminoethyl)-3,4,6,7-tetrahydro-2H-pyrimido[6,1-a]isoquinolin-4-one (RPL554); and
(ii) a diluent which is 1,1,1,2-tetrafluoroethane (HFA-134a),
wherein the liquid pharmaceutical composition contains less than 0.05 % by weight of a surfactant relative to the total weight of the composition.

2. A liquid pharmaceutical composition for use according to claim 1, wherein the liquid pharmaceutical composition contains less than 0.05 % by weight of any additional excipient selected from a co-solvent and a surfactant relative to the total weight of the composition.

3. A liquid pharmaceutical composition for use according to claim 1 or claim 2, wherein the liquid pharmaceutical composition contains less than 0.05 % by weight of any additional excipient relative to the total weight of the composition.

4. A liquid pharmaceutical composition for use according to any one of the preceding claims, wherein the liquid pharmaceutical composition contains less than 0.01 % by weight of said surfactant or said additional excipient, relative to the total weight of the composition.

5. A liquid pharmaceutical composition for use according to any one of the preceding claims, wherein the liquid pharmaceutical composition contains less than 0.001 % by weight of said surfactant or said additional excipient, relative to the total weight of the composition.

6. A liquid pharmaceutical composition for use according to any one of the preceding claims, wherein the particles comprise at least 50% by weight of RPL554 relative to the total weight of the particles.

7. A liquid pharmaceutical composition for use according to any one of the preceding claims, wherein the particles comprise at least 99% by weight of RPL554 relative to the total weight of the particles.

8. A liquid pharmaceutical composition for use according to any one of the preceding claims, wherein RPL554 is the sole active agent.

9. A liquid pharmaceutical composition for use according to any one of the preceding claims which comprises at least 99.5 % by weight of (i) RPL554 and (ii) 1,1,1,2-tetrafluoroethane relative to the total weight of the composition.

10. A liquid pharmaceutical composition for use according to any one of the preceding claims, wherein the particles comprising RPL554 have a particle size distribution with a Dv50 (median particle size by volume) value of from 0.2 µm to 5 µm.

11. A liquid pharmaceutical composition for use according to any one of the preceding claims, wherein the concentration of particles comprising RPL554 in the liquid pharmaceutical composition is from 0.1 mg/mL to 200 mg/mL.

12. A liquid pharmaceutical composition for use according to any one of the preceding claims which is suitable for administration by pressurised metered dose inhaler.

13. A liquid pharmaceutical composition for use according to any one of the preceding claims, which liquid pharmaceutical composition is in a pressurised metered dose inhaler.

## Patentansprüche

1. Flüssige pharmazeutische Zusammensetzung für die Verwendung bei der Behandlung oder Vorbeugung von chronisch obstruktiver Lungenerkrankung (COPD),
wobei die flüssige pharmazeutische Zusammensetzung für die Verabreichung durch Inhalation geeignet ist und umfasst:
(i) eine Suspension von Teilchen, umfassend 9,10-Dimethoxy-2-(2,4,6-trimethylphenylimino)-3-(N-carbamoyl-2-aminoethyl)-3,4,6,7-tetrahydro-2H-pyrimido[6,1-a]isochinolin-4-on (RPL554); und
(ii) ein Verdünnungsmittel, das 1,1,1,2-Tetrafluorethan (HFA-134a) ist,
wobei die flüssige pharmazeutische Zusammensetzung weniger als 0,05 Gew.-% eines Tensids, bezogen auf das Gesamtgewicht der Zusammensetzung, enthält.

2. Flüssige pharmazeutische Zusammensetzung für die Verwendung nach Anspruch 1, wobei die flüssige pharmazeutische Zusammensetzung weniger als 0,05 Gew.-% eines beliebigen zusätzlichen Hilfsstoffs, ausgewählt aus einem Co-Lösungsmittel und einem Tensid, bezogen auf das Gesamtgewicht der Zusammensetzung, enthält.

3. Flüssige pharmazeutische Zusammensetzung für die Verwendung nach Anspruch 1 oder 2, wobei die flüssige pharmazeutische Zusammensetzung weniger als 0,05 Gew.-% eines beliebigen zusätzlichen Hilfsstoffs, bezogen auf das Gesamtgewicht der Zusammensetzung, enthält.

4. Flüssige pharmazeutische Zusammensetzung für die Verwendung nach einem der vorhergehenden Ansprüche, wobei die flüssige pharmazeutische Zusammensetzung weniger als 0,01 Gew.-% des Tensids oder des zusätzlichen Hilfsstoffs, bezogen auf das Gesamtgewicht der Zusammensetzung, enthält.

5. Flüssige pharmazeutische Zusammensetzung für die Verwendung nach einem der vorhergehenden Ansprüche, wobei die flüssige pharmazeutische Zusammensetzung weniger als 0,001 Gew.-% des Tensids oder des zusätzlichen Hilfsstoffs, bezogen auf das Gesamtgewicht der Zusammensetzung, enthält.

6. Flüssige pharmazeutische Zusammensetzung für die Verwendung nach einem der vorhergehenden Ansprüche, wobei die Teilchen mindestens 50 Gew.-% RPL554, bezogen auf das Gesamtgewicht der Teilchen, umfassen.

7. Flüssige pharmazeutische Zusammensetzung für die Verwendung nach einem der vorhergehenden Ansprüche, wobei die Teilchen mindestens 99 Gew.-% RPL554, bezogen auf das Gesamtgewicht der Teilchen, umfassen.

8. Flüssige pharmazeutische Zusammensetzung für die Verwendung nach einem der vorhergehenden Ansprüche, wobei RPL554 der einzige Wirkstoff ist.

9. Flüssige pharmazeutische Zusammensetzung für die Verwendung nach einem der vorhergehenden Ansprüche, die mindestens 99,5 Gew.-% (i) RPL554 und (ii) 1,1,1,2-Tetrafluorethan, bezogen auf das Gesamtgewicht der Zusammensetzung, umfasst.

10. Flüssige pharmazeutische Zusammensetzung für die Verwendung nach einem der vorhergehenden Ansprüche, wobei die RPL554 umfassenden Teilchen eine Teilchengrößenverteilung mit einem Dv50- (mediane Teilchengröße nach Volumen) Wert von 0,2 µm bis 5 µm aufweisen.

11. Flüssige pharmazeutische Zusammensetzung für die Verwendung nach einem der vorhergehenden Ansprüche, wobei die Konzentration der RPL554 umfassenden Partikel in der flüssigen pharmazeutischen Zusammensetzung von 0,1 mg/ml bis 200 mg/ml beträgt.

12. Flüssige pharmazeutische Zusammensetzung für die Verwendung nach einem der vorhergehenden Ansprüche, die für die Verabreichung durch ein unter Druck stehendes Dosieraerosol geeignet ist.

13. Flüssige pharmazeutische Zusammensetzung für die Verwendung nach einem der vorhergehenden Ansprüche, wobei sich die flüssige pharmazeutische Zusammensetzung in einem unter Druck stehenden Dosieraerosol befindet.

## Revendications

1. Composition pharmaceutique liquide à utiliser dans le traitement ou la prévention de la maladie pulmonaire obstructive chronique (MPOC),
laquelle composition pharmaceutique liquide convient à l'administration par inhalation et comprend:
(i) une suspension de particules comprenant du 9,10-diméthoxy-2-(2,4,6-triméthylphénylimino)-3-(N-carbamoyl-2-aminoéthyl)-3,4,6,7-tétrahydro-2H-pyrimido [6,1-a]isoquinolin-4-one (RPL554) ; et
(ii) un diluant qui est le 1,1,1,2-tétrafluoroéthane (HFA-134a), dans laquelle la composition pharmaceutique liquide contient moins de 0,05% en poids d'un tensioactif par rapport au poids total de la composition.

2. Composition pharmaceutique liquide à utiliser selon la revendication 1, dans laquelle la composition pharmaceutique liquide contient moins de 0,05% en poids de tout excipient supplémentaire choisi parmi un co-solvant et un tensioactif par rapport au poids total de la composition.

3. Composition pharmaceutique liquide à utiliser selon la revendication 1 ou la revendication 2, dans laquelle la composition pharmaceutique liquide contient moins de 0,05% en poids de tout excipient supplémentaire par rapport au poids total de la composition.

4. Composition pharmaceutique liquide à utiliser selon l'une quelconque des revendications précédentes, dans laquelle la composition pharmaceutique liquide contient moins de 0,01% en poids dudit tensioactif ou dudit excipient supplémentaire, par rapport au poids total de la composition.

5. Composition pharmaceutique liquide à utiliser selon l'une quelconque des revendications précédentes, dans laquelle la composition pharmaceutique liquide contient moins de 0,001% en poids dudit tensioactif ou dudit excipient supplémentaire, par rapport au poids total de la composition.

6. Composition pharmaceutique liquide à utiliser selon l'une quelconque des revendications précédentes, dans laquelle les particules comprennent au moins 50% en poids de RPL554 par rapport au poids total des particules.

7. Composition pharmaceutique liquide à utiliser selon l'une quelconque des revendications précédentes, dans laquelle les particules comprennent au moins 99% en poids de RPL554 par rapport au poids total des particules.

8. Composition pharmaceutique liquide à utiliser selon l'une quelconque des revendications précédentes, dans laquelle RPL554 est le seul agent actif.

9. Composition pharmaceutique liquide à utiliser selon l'une quelconque des revendications précédentes qui comprend au moins 99,5% en poids de (i) RPL554 et (ii) 1,1,1,2-tétrafluoroéthane par rapport au poids total de la composition.

10. Composition pharmaceutique liquide à utiliser selon l'une quelconque des revendications précédentes, dans laquelle les particules comprenant le RPL554 ont une distribution granulométrique avec une valeur Dv50 (granulométrie moyenne en volume) de 0,2 µm à 5 µm.

11. Composition pharmaceutique liquide à utiliser selon l'une quelconque des revendications précédentes, dans laquelle la concentration de particules comprenant le RPL554 dans la composition pharmaceutique liquide est de 0,1 mg/mL à 200 mg/mL.

12. Composition pharmaceutique liquide à utiliser selon l'une quelconque des revendications précédentes, qui est appropriée pour une administration par un inhalateur doseur pressurisé.

13. Composition pharmaceutique liquide à utiliser selon l'une quelconque des revendications précédentes, laquelle composition pharmaceutique liquide est dans un inhalateur doseur pressurisé.
